**Europäisches Patentamt**

**European Patent Office**

**Office européen des brevets**

(19)

(11) Veröffentlichungsnummer: **0 172 504 B1**

(12) **EUROPÄISCHE PATENTSCHRIFT**

(45) Veröffentlichungstag der Patentschrift: **03.04.91**

(51) Int. Cl.⁵: **C12M 1/00, C12P 1/00, C12M 1/40**

(21) Anmeldenummer: **85109996.0**

(22) Anmeldetag: **08.08.85**

(54) **Biologischer Reaktor.**

(30) Priorität: **18.08.84 DE 3430488**

(43) Veröffentlichungstag der Anmeldung:
**26.02.86 Patentblatt 86/09**

(45) Bekanntmachung des Hinweises auf die
Patenterteilung:
**03.04.91 Patentblatt 91/14**

(84) Benannte Vertragsstaaten:
**DE FR GB**

(56) Entgegenhaltungen:
**FR-A- 2 495 843**

**CHEMICAL ABSTRACTS, Band 86, 1977, Seite 550, Zusammenfassung Nr. 80763q, Columbus, Ohio, US; F. ROKURO: "Formation of bisulfate lamellar compounds in graphite fiber electrodes"**

**BIOTECHNOLOGY AND BIOENGINEERING, Band 17, 1975, Seiten 1379-1382, John Wiley & Sons, Inc., US; R.W. COUGHLIN et al.: "Simplified flow reactor for electrochemically driven enzymatic reactions involving cofactors"**

(73) Patentinhaber: **BASF Aktiengesellschaft
Carl-Bosch-Strasse 38
W-6700 Ludwigshafen(DE)**

(72) Erfinder: **Habermann, Wolfgang
Gonsenheimer Spiess 8
W-6500 Mainz 1(DE)**
Erfinder: **Pommer, Ernst-Heinrich, Dr.
Berliner Platz 7
W-6703 Limburgerhof(DE)**
Erfinder: **Hammes, Peter, Dr.
Haagweg 14
W-6701 Ruppertsberg(DE)**

BIOTECHNOLOGY AND BIOENGINEERING, Band 17, 1975, Seiten 515-526, John Wiley & Sons, Inc., US; R.W. COUGHLIN et al.: "Immobilized-Enzyme continuous-flow reactor incorporating continuous electrochemical regeneration of NAD"

CHEMICAL ABSTRACTS, Band 88, 1978, Seiten 214-215, Zusammenfassung Nr. 46811k, Columbus, Ohio, US; S.D. VARFOLOMEEV et al.: "Bioelectrocatalysis. Hydrogenase as catalyst of electrochemical hydrogen ionization"

CHEMICAL ABSTRACTS, Band 103, Nr. 10, 1985, Seite 462, Zusammenfassung Nr. 78202g, Columbus, Ohio, US; K. TAKAMURA et al.: "Anodically pretreated carbon electrode surfaces and their effects on the electrode reaction"

CHEMICAL ABSTRACTS, Band 94, 1981, Seite 349, Zusammenfassung Nr. 98843r, Columbus, Ohio, US; & SU-A-593 439 (INSTITUTE OF CHEMICAL SCIENCES, ACADEMY OF SCIENCES, KAZAKH S.S.R.) 15-10-1980

## Beschreibung

Die vorliegende Erfindung betrifft einen biologischen Reaktor, der mit Mikroorganismen und/oder Enzymen betrieben wird, zur oxidativen Umsetzung von organischen Verbindungen, bei dem man die umzusetzenden organischen Verbindungen in Gegenwart von Biokatalysatoren im Reaktorraum einsetzt und die Enzyme an einer polarisierten Elektrode regeneriert, wobei die Polarisation der Elektrode über eine durch einen Polyelektrolyten vom Reaktorraum separierte, in einem Elektrolytraum befindliche Katalysatoreleketrode, die durch ein Oxydationsmittel polarisiert wird, erfolgt.

Bei technischen mikrobiellen Verfahren, die aerob ablaufen, verwendet man Reaktoren, die zur Regeneration der Enzyme oder Beatmung der Mikroorganismen mit Sauerstoff bzw. Luft betrieben werden. Um einen ausreichenden Sauerstoffeintrag zu erzielen, muß man Strahldüsen-, Tauchstrahl- und Rührsysteme einsetzen. Durch intensive Mischung müssen ständig neue Phasengrenzflächen geschaffen werden, um einen guten Stoffaustausch zu erreichen, da nur der gelöste Sauerstoff an der Reaktion teilnimmt. Bei dieser Art des Sauerstoffeintrags entstehen hohe Scherkräfte, die eine zumindest teilweise Schädigung der Mikroorganismen und des Enzymsystems zur Folge haben. Ein weiteres Problem ist die Schaumbekämpfung bei der Begasung. Hier müssen oft teure, nicht metabolisierbare Antischaummittel eingesetzt werden, die zum Teil im gewünschten Produkt verbleiben. Es wurden deshalb schon mechanische Schaumzerstörer eingesetzt, welche aber wiederum Schädigungen der Mikroorganismen bedingten. Ein weiteres Problem ist die Sterilität der zugeführten Luft, die bei empfindlichen Produkten oder Mikroorganismen frei von Fremdkeimen sein muß.

Es war daher Aufgabe der vorliegenden Erfindung einen biologischen Reaktor zu entwickeln der eine einfache, effektivere Regeneration der Biokatalysatoren ohne deren Schädigung ermöglicht, und Techniken zur Verbesserung der Trennung von Oxidationsmittel, umzusetzenden organischen Verbindungen sowie der Biokatalysatoren zu finden.

Diese Aufgabe wurde erfindungsgemäß dadurch gelöst, daß zur Steuerung des biochemischen Prozesses an Stelle von Sauerstoff im Reaktorraum eine anodisch polarisierte Elektrode aus einem bei Potentiallagen $\epsilon_h$ von + 1,3 V bis + 10 V in wäßrigen, sauerstoffhaltigen Mineralsäuren oberflächlich anoxidierten, elektrisch leitenden Kohlenstoffmaterial eingesetzt wird.

Weitere vorteilhafte Ausgestaltungen des Erfindungsgedankens sind Gegenstand der Unteransprüche.

Die Erfindung wird nachstehend anhand der Zeichnungen näher erläutert.

Figur 1 zeigt die Polarisation einer Elektrode 1 durch eine Katalysatorelektrode 2, an der Oxidationsmittel reduziert werden, wobei die beiden Elektroden 1, 2 niederohmig verbunden sind.

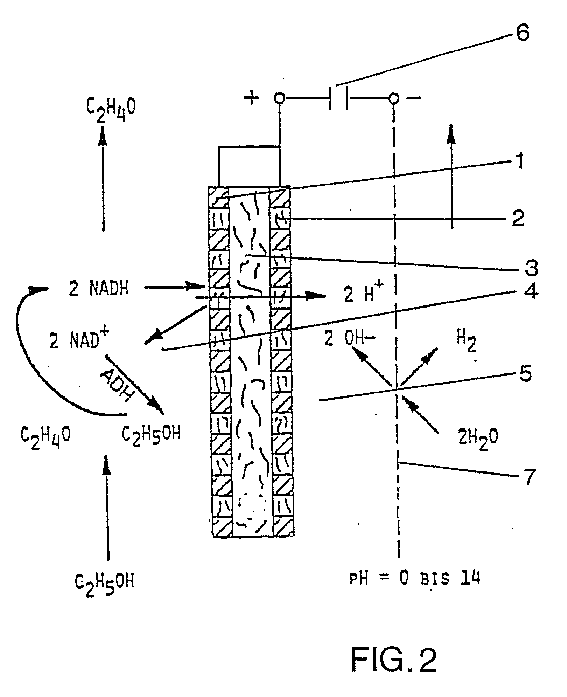

Figur 2 zeigt die Polarisation der Elektrode 1, die mit der Katalysatorelektrode 2 niederohmig verbunden ist, über eine äußere Spannungsquelle 6 gegen eine Hilfskathode 7.

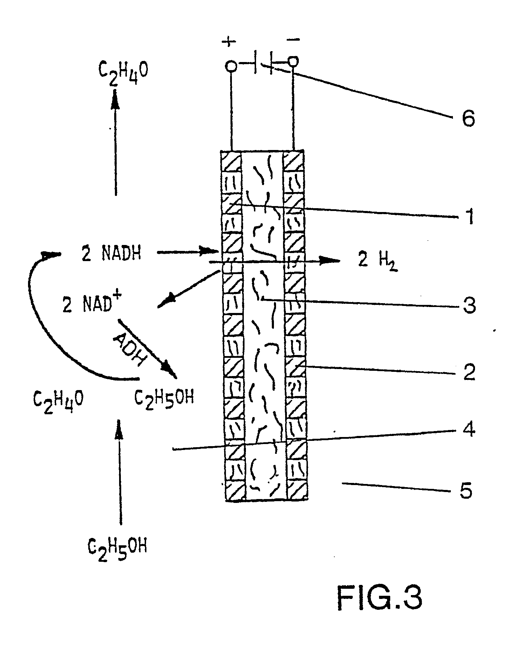

Figur 3 zeigt die Polarisation der Elektrode 1 gegen die Katalysatorelektrode 2 über eine äußere Spannungsquelle 6.

Die Figuren 4 bis 6 zeigen verschiedene Ausführungsformen von Rohrreaktoren.

Eine anodisch polarisierte Elektrode 1 wird auf einem Polyelektrolyten 3 (Ionenaustauscher) fixiert, der einen Reaktorraum 4 von einem Elektrolytraum 5 trennt, wobei sich auf der Oberfläche oder in der Nähe der Oberfläche des Polyelektrolyten 3 eine zweite polarisierbare, ionendurchlässige Katalysatorelektrode 2 befindet, welche elektrisch leitend oder über eine äußere Spannungsquelle 6 mit der anodisch polarisierten Elektrode 1 im Reaktorraum 4 verbunden ist.

Das anodisch polarisierte Kohlenstoffmaterial der Elektrode 1 besteht beispielsweise aus Graphit, Graphitwolle, Anthrazit oder Aktivkohle, das vorzugsweise elektrochemisch in wäßrigen, sauerstoffhaltigen Mineralsäuren wie z.B. Salpetersäure, Phosphorsäure, Schwefelsäure und Perchlorsäure bei Potentiallagen $\epsilon_h \geq$ + 1,3 V anoxidiert, wobei die Potentiale $\epsilon_h$-Werte von + 10 V nicht übersteigen sollten, und anschließend durch kathodische Reduktion in Mineralsäuren aktiviert wurden. Bevorzugt arbeitet man in wäßriger Salpetersäure bei Oxidationspotentialen von $\epsilon_h$ = + 1,8 bis + 2,5 Volt.

Zur anodischen Oxidation setzt man 2 bis 80 gew.%ige, vorzugsweise 30 bis 65 gew.%ige wäßrige Salpetersäure ein. Die Oxidation erfolgt bei Temperaturen < + 100 °C, vorzugsweise bei + 10 bis + 40 °C und Stromdichten von 0,1 bis 10 KA/m², vorzugsweise 0,5 bis 4 KA/m² Elektrodenoberfläche.

Anstelle von Salpetersäure kann auch wäßrige Schwefelsäure, Phosphorsäure oder Perchlorsäure eingesetzt werden. Nach der anodischen Oxidation wird das Elektrodenmaterial durch eine kathodische Reduktion in wäßriger Schwefelsäure von Oxidationsmittelresten befreit. Zur kathodischen Reduktion wird vorzugsweise 5 bis 20 gew.%ige wäßrige Schwefelsäure verwendet.

Weiterhin kann die Oxidation der Graphitoberfläche, wenn auch wesentlich weniger effektiv, chemisch mit Stickoxiden, bevorzugt Stickstoffdioxid, bei Temperaturen zwischen + 300 °C und + 800 °C oder in chromathaltigen Mineralsäuren, wie z.B. chromathaltige Schwefelsäure, Phosphorsäure oder Perchlorsäure erfolgen.

Die Polarisation der Katalysatorelektrode 2 im Elektrolytraum 5 erfolgt mit einem Oxidationsmittel wie z.B. Sauerstoff, Wasserstoffperioxid, Perverbindungen, Chlor, Salpetersäure, Eisen-III-Salzen, Cer-III-salzen, Chrom-VI-salzen, sauren Vanadaten oder nitrosen Gasen, die im Elektrolytraum 5 in hoher Konzentration eingesetzt werden können. Die Umsetzung der Oxidantien findet an der Oberfläche der Katalysatorelektrode 2 statt, die im Elektrolytraum 5 fixiert ist und gute Elektronenaustauschbedingungen aufweist, während die Regeneration des Enzymsystems und/oder oder Mikroorganismen durch Elektronenaustausch mit der im Reaktorraum 4 befindlichen Katalysatorelektrode 1 erfolgt.

Bei Polarisation der Katalysatorelektrode 1 über eine Hilfselektrode 7 ist außerdem die Überspannung des Katalysatorelektrodenmaterials im Elektrolytraum 5 und die Stromdichte maßgebend.

Als Polyelektrolyte 3, die den Träger des Katalysatorelektrodenmaterials und den Separator zwischen Reaktorraum 4 und Oxidationsraum 5 darstellen, werden homogene und heterogene Kationen- oder Anionenaustauschermembranen eingesetzt. Bevorzugt sind Ionenaustauschermembranen auf Basis von Polymeren oder Copolymeren aus Styrol, Styrol und Divinylbenzol, Vinylchlorid und Acrylnitril, Olefinen, perfluorierten Olefinen die als ladungstragende Gruppen quartäre Ammonium-, Sulfonsäure-, oder Carboxlygruppen tragen. Besonders geeignet sind hochvernetzte Ionenaustauschermaterialien mit einer geringen Quellfähigkeit, und hoher Konzentration an ladungstragenden Gruppen, die kaum eine Durchlässigkeit für Coionen aufweisen.

Die polarisierte Elektrode 1 und die Katalysatorelektrode 2 werden z.B. in Form von Netzen, Streckmetallgittern, Sinterkörpern, Lochblechen, Gitterrosten, Schwämmen und dgl. ausgeführt, wobei die freie Fläche etwa 15 bis 85 %, vorzugsweise 25 bis 65 % der Elektrodenfläche beträgt, so daß ein ausreichender Ionenfluß stattfinden kann. Bei dieser Ausführung ist zumindest die polarisierte Elektrode 1 oberflächlich mit einem anoxidiertem, elektrisch leitenden Kohlenstoffmaterial beschichtet.

Zur Verbesserung der Leitfähigkeit ist es günstig, die Poren, Kanäle und Lochstellen zumindest teilweise mit Ionenaustauschermaterial auszufüllen. Hierzu kann man z.B. feinkörnige Ionenaustauscher mit einem Bindemittel oder Polystyrol mit einem Molgewicht 50.000 einarbeiten, in das z.B. durch Chlormethylierung und Aminbehandlung quarternäre Ammoniumgruppen oder durch Sulfierung Sulfonsäuregruppen eingebaut werden. Bei einem derartigen Aufbau ist die Leitfähigkeit des Reaktorraummediums nicht mehr von Bedeutung.

Als Materialien für die Katalysatorelektroden 2 kann man im Elektrolytraum 5 Titan, Zirkon, Vanadium, Tantal, Niob, Nickel oder Graphit als Träger einsetzen, welches oberflächlich mit Platinmetallen wie z.B. Iridium, Rhodium, Ruthenium, Platin, Palladium, deren Oxiden, Silber, Gold, Kobalt-Aluminiumspinellen, mit dotierten Lanthankobaltid, sauerstoffübertragenden Kobalt-, Mangan-, Eisenphthalocyaninen, Eisendibenzo-tetra-aza-annulen, Kobalt-II-bis (salicylidenethylendiaminen) oder Kobalt-tetra-aza-annulen aktiviert ist. Diese Aktivatoren können einzeln oder als Kombination mehrerer Aktivatoren verwendet werden.

Bevorzugt geeignet sind im Elektrolytraum 5 Katalysatorelektroden 2 aus Graphit oder mit Graphit beschichteten Trägermaterialien, die anodisch in wäßriger Salpetersäure anoxidiert und anschließend mit Molybdän- und/oder Wolfram-VI-oxiden und Alkalisulfiden oder Molybdän-VI-oxiden und Iodaten dotiert wurden. Diese Elektrodenmaterialien können zusätzlich elektronenleitende und ionenleitende Substanzen enthalten. Anstelle von Molybdän- und/oder Wolfraum-VI-oxiden können auch anreduzierte Vanadium-V-oxiverbindungen, wie Vanadin-V-oxid, Vanadin-IV-oxid, Tellur-VI-Verbindungen, wie Tellursäure, Tellurtrioxid und Selen-VI-Verbindungen, wie z.B. Selensäure, Selentrioxid eingesetzt werden.

Weiterhin geeignet sind im Elektrolytraum 5 Elektroden 2 aus Titan, mit Beschichtungen aus metallischen oder oxidischem Iridium, Platin, Rhodium, Palladium, Gold und Silber in Kombination mit Titansuboxid oder Titancarbid sowie Beschichtungen aus 4-tert.-Alkyl-Eisen- oder Manganphthalocyaninen oder sulfonierten Produkten davon, die in Kombination mit ionenaustauschenden und elektronenleitenden Substanzen oder ionen- und elektronenleitenden Substanzen enthaltenden Bindemitteln aufgetragen sind.

Zur Verbesserung der Elektronenaustauschbedingungen an der polarisierten Elektrode 1 und zur Einsparung von Enzymen werden an dieser oberflächlich anoxidierten Elektrode 1 durch Sorption Enzyme und Mikroorganismen immobilisiert. Die sorptive Immobilisierung erfolgt vorzugsweise in enzym- und/ oder mikroorganismenhaltigen Elektrolytpuffern, wie z.B. Phosphatpuffer lösungen. Weiterhin eignen sich als Träger für die Enzyme und Mikroorganismen auf der polarisierten Elektrode 1, z.B. Nickel-Nickeloxid, Titansuboxid, Zeolithe, Sepharose, Cyanurchlorid-Graphit, Carbodiimid-Graphit, poröse Copolymerisate aus Styrol- und Divinylbenzol sowie makroporöse organische Kationen- und Anionenaustauscher wie beispielsweise Polymere und Copolymere aus Styrol und Divinylbenzol, Vinylchlorid und Acrylnitril, Olefine, perfluorierte Olefine, die als ladungstragende Gruppen quartäre Ammonium-, Sulfonsäure- oder Carboxylgruppen tragen. Vorteilhaft ist es, die enzymtragenden Verbindungen mit ionen- und elektronenleitenden organischen oder anorganischen Substanzen auf der Elektrodenoberfläche zu fixieren.

Bei Einsatz von Hilfskathoden verwendet man z.B. Eisen, Nickel, Kobalt, Graphit sowie Chrom-Nickelstähle als Elektrodenmaterial.

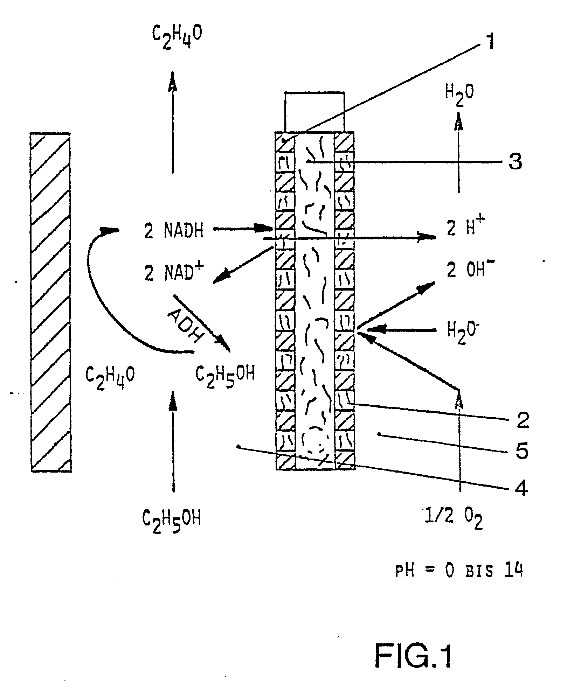

Figur 1 zeigt eine Brennstoffzelle zur Enzymregenerierung mit einer Sauerstoffreduktionselektrode als Katalysatorelektrode 2, wobei die polarisierte Elektrode 1 niederohmig mit der Katalysatorelektrode 2 verbunden ist. Der Reaktorraum 4 (Anodenraum) und der Elektrolytraum 5 (Kathodenraum) ist durch einen Polyelektrolyten 3 getrennt. Beide Elektroden sind auf der Polyelektrolytoberfläche 3 (Ionenaustauscher) fixiert. Beim Betrieb der Zelle wird das reduzierte Enzym (NAD) regeneriert. An der Katalysatorelektrode 2 werden äquivalente Mengen Sauerstoff reduziert.

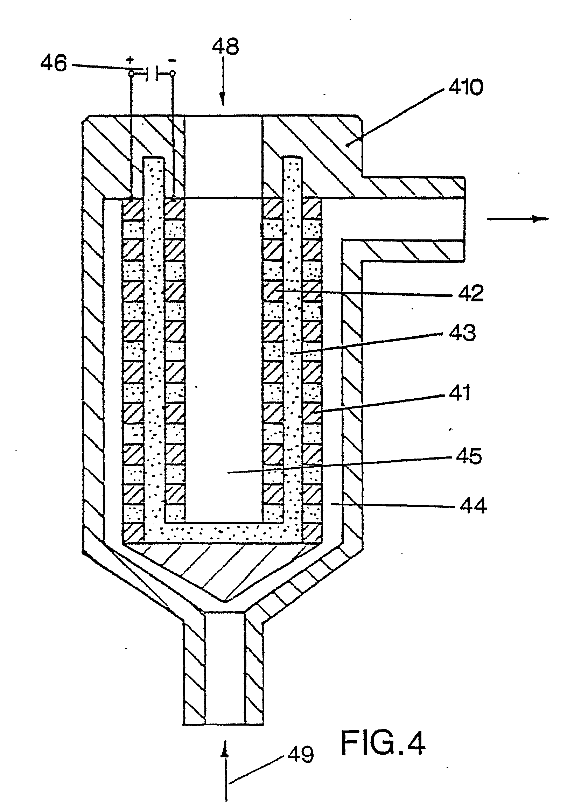

Figur 4 zeigt einen Rohrreaktor, bei dem die Elektrode 41 und die Katalysatorelektrode 42, jeweils in Streckmetallausführung auf dem Polyelektrolyten 43 fixiert sind. Die Elektrode 41 wird über eine äußere Spannungsquelle 46 gegen die Katalysatorelektrode 42 polarisiert. Die Reaktorwandung 410 besteht aus Polypropylen. Der Elektrolytraum 45 mit dem Elektrolyten 48 ist vom Reaktorraum 44 mit dem Reaktionsmedium 49 durch den Polyelektrolyten 43 getrennt.

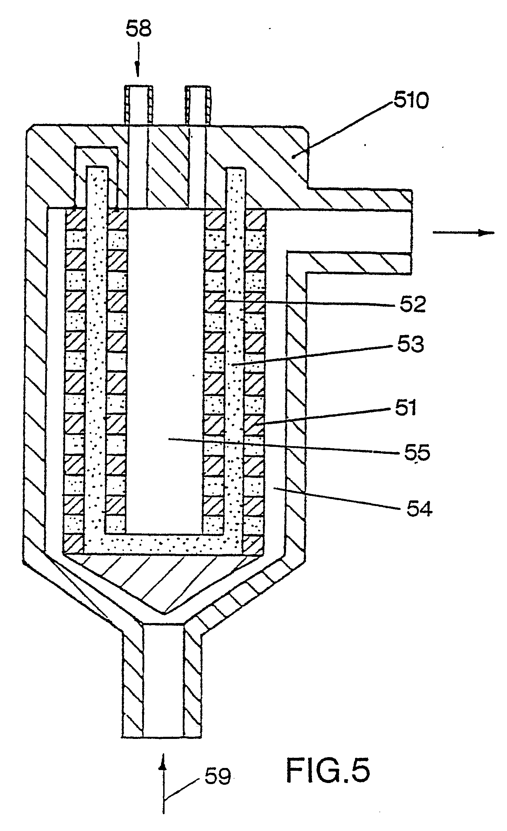

Figur 5 zeigt einen Rohrreaktor, bei dem die Elektrode 51 mit dem Katalysatorelektrode 52 niederohmig verbunden ist. Beide Elektroden 51, 52 sind in Streckmetallausführung auf dem Polyelektrolyten 53 fixiert. Der Elektrolytraum 55, welcher mit dem Oxidationsmittel 58 betrieben wird, ist vom Reaktorraum 54 mit dem Reaktionsmedium 59 durch den Polyelektrolyten 53 separiert. Die Reaktorwandung 510 besteht ebenfalls aus Polypropylen.

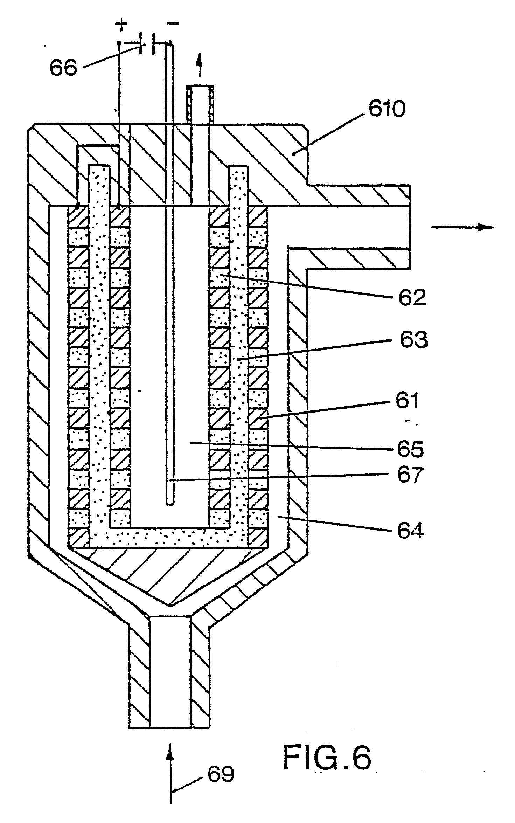

Figur 6 zeigt einen Rohrreaktor, bei dem die Elektrode 61, mit der Katalysatorelekrode 62 niederohmig verbunden ist. Die Katalysatorelektrode 62 wird gegen eine Hilfskathode 67 über eine äußere Spannungsquelle 66 pola risiert. Der Elektrolytraum 65 ist vom Reaktorraum 64 mit dem Reaktionsmedium 69 durch den Polyelektrolyten 63 getrennt. Die Reaktorwandung 610 besteht aus Polyethylen.

Beispiel 1

Ein Rohrreaktor, der gemäß Figur 5 aufgebaut ist, enthält zur Trennung von Reaktor- 54 und Elektrolytraum 55 sowie als Träger für die polarisierte Elektrode 51 einen Polyelektrolyten 53, der durch Copolymerisation von Tetrafluorethylen mit ungesättigten perfluorierten Ethern, die Sulfonsäurefluoridgruppen enthalten hergestellt wurde. Die Abmessungen des Polyelektrolyten 53 betragen 30 mm Durchmesser, 220 mm Länge und 0,85 mm Wandstärke. Auf der im Reaktorraum 54 befindlichen Polyelektrolytseite ist ein Titanstreckmetall mit einer Maschenweite von 6 x 13 mm, einer Stegbreite von 2,5 mm, einer wirksamen Oberfläche von 1,98 $m^2/m^2$ geometrischer Oberfläche und einem freien Querschnitt von 36 % fixiert, welches oberflächlich mit Graphit beschichtet ist. Die Oberfläche des Graphits wurde durch anodische Oxidation bei 2 KA/$m^2$ in 50 gew.%iger wäßriger Salpetersäure und anschließende kathodische Reduktion in 10 %iger Schwefelsäure aktiviert. Im Elektrolytraum 55 befindet sich auf der Polyelektrolytoberfläche 53 ein Titanstreckmetall von 7 x 12,5 mm Maschenweite, 2 mm Stegbreite, einer wirksamen Oberfläche von 1,82 $m^2/m^2$ geometrischer Oberfläche und einem freien Querschnitt von 33 %. Dieses Streckmetall ist mit in 50 %iger wäßriger Salpetersäure anodisch anoxidiertem Graphit beschichtet und hydratisiertem Mo-VI-oxid sowie $K_2S$ dotiert. Die beiden Katalysatorelektroden (Streckmetalle) sind durch einen Titanbügel niederohmig verbunden. Der Abstand der Katalysatorelektrodenoberfläche im Reaktorraum zur Reaktorwandung beträgt 3 mm um einen guten Stoffumsatz zu gewährleisten. Der Elektrolytraum 55 wird mit 5 %iger wäßriger Schwefelsäure gefüllt, der 0,4 Gew.% Eisen-III-nitrat eingesetzt werden. Als Oxidationsmittel 58 wird in den Elektrolytraum 55 Sauerstoff eingetragen. Der Reaktorraumspalt wird von dem Reaktionsmedium, z.B. Rubensirup, mit einer Geschwindigkeit von 0,3 bis 2,5 m/sec durchströmt, wobei die Enzyme an der polarisierten Elektrode 51 regeneriert bzw. die Mikroorganismen beatmet werden. Einsetzen kann man den Reaktor zur Herstellung von Glutaminsäure aus Stärke, Rohr- oder Rübensirup, wenn man als Mikroorganismen Mutanten von Corynebakterium glutamicum verwendet.

Beispiel 2

Ein Rohrreaktor der gemäß Figur 5 aufgebaut ist, enthält zur Trennung von Reaktorraum 54 und Elektrolytraum 55 sowie als Träger für die Elektroden 51, 52 einen Polyelektrolyten 53, der durch Copolymerisation von Tetrafluorethylen mit ungesättigten perfluorierten Ethern, die Sulfonsäurefluoridgruppen und Carbonsäureester enthalten, hergestellt wurde. Auf der im Reaktorraum 54 befindlichen Polyelektrolytseite ist ein platiniertes Titanstreckmetall mit einer Maschenweite von 6 x 13 mm, einer Stegbreite von 2,5 mm, einer wirksamen Oberfläche von 1,98 $m^2/m^2$ geomtrischer Oberfläche und einem freien Querschnitt von 36 % fixiert. Die Oberfläche des Streckmetalls ist mit 0,5 bis 0,8 mm starker Graphitwolle abgedeckt, die durch Temperung von Baumwolle bei +2500°C in einer Inertgasatmosphäre erhalten wurde. Oxidiert wurde die Graphitwolle in 50 gew.%iger wäßriger Salpetersäure bei einer anodischen Stromdichte von 2 KA/m$^2$ und Temperaturen von $+26°$ C. Das Oxidationspotential $\epsilon_h$ betrug $\sim +2,12$ V. Salpetersäurereste wurden nach der Oxidation durch kathodische Reduktion in 10 %iger wäßriger Schwefelsäure entfernt. Im Elektrolytraum 55 ist auf der Polyelektrolytoberfläche 53 ein platiniertes Titanstreckmetall mit Graphitwolle wie im Reaktorraum angebracht. Die Graphitwolle ist mit hydratisiertem Vanadiumpentoxid imprägniert, welches zusätzlich Titan-IV-oxid und Titanvanadat enthält. Beide Elektroden 51, 52 sind niederohmig durch einen Titanbügel verbunden. Der Elektrolytraum 55 wird mit 5 gew.%iger Schwefelsäure, die 1 Gew.% Eisen-III-sulfat enthält, gefüllt und mit Sauerstoff begast. Die Elektrode 51 im Reaktorraum 54 wird mit einem Phosphatpuffer vom pH-Wert = 7, der 1 Mol Alkoholdehydrogenase ADH aus Leuconostoc enthält, imprägniert, so daß ADH auf der Oberfläche der Graphitwolle immobilisiert wird. An der polarisierten Elektrode 51 im Reaktorraum 54 können beim Betrieb des Reaktors Alkohole zu Aldehyden umgesetzt werden, ohne daß das Coenzym Nicotinamid-adenin-dinucleotid NAD benötigt wird.

Die Elektrode 51 kann aber auch zusätzlich mit Coenzymen imprägniert sein.

An Stelle von Luftsauerstoff kann auch Wasserstoffperoxid als Oxidans im Elektrolytraum 55 eingesetzt werden. In diesem Falle wird auf den Zusatz von Eisen-III-Ionen verzichtet.

## Ansprüche

1. Biologischer Reaktor, der mit Mikroorganismen und/oder Enzymen betrieben wird, zur oxidativen Umsetzung von organischen Verbindungen, bei dem man die umzusetzenden organischen Verbindungen in Gegenwart von Biokatalysatoren im Reaktorraum (4) (Anodenraum) einsetzt und die Enzyme an einer polarisierten Elektrode (1) regeneriert, wobei die Polarisation der Elektrode (1) über eine durch einen Polyelektrolyten (3) vom Reaktorraum (4) separierte, in einem Elektrolytraum (5) befindliche Katalysatorelektrode (2), die durch ein Oxydationsmittel polarisiert wird, erfolgt, **dadurch gekennzeichnet,** daß die Elektrode (1) aus einem, bei Potentiallagen $\epsilon_h$ von $+1,3$ V bis $+10$ V in wäßrigen, sauerstoffhaltigen Mineralsäuren oberflächlich anoxidierten, elektrisch leitenden Kohlenstoffmaterial hergestellt ist.

2. Biologischer Reaktor nach Anspruch 1, **dadurch gekennzeichnet,** daß die anodisch polarisierte Elektrode (1) auf einem Polyelektrolyten (3) (Ionenaustauscher) fixiert ist, der den Reaktorraum (4) vom Elektrolytraum (5) trennt, und wobei sich auf der Oberfläche oder in der Nähe der Oberfläche des Polyelektrolyten (3) eine zweite polarisier-bare, ionendurchlässige Katalysatorelektrode (2) befindet, welche elektrisch leitend oder über eine äußere Spannungsquelle (6) mit der anodisch polarisierten Elektrode (1) im Reaktorraum (4) verbunden ist.

3. Biologischer Reaktor nach Anspruch 1 und 2, **dadurch gekennzeichnet,** daß die anodisch polarisierte Elektrode (1) aus Graphit, Graphitwolle oder Aktivkohle besteht, die elektrochemisch in wäßriger Salpetersäure anoxidiert und anschließend durch kathodische Reduktion in Mineralsäuren aktiviert wurde.

4. Biologischer Reaktor nach Anspruch 1 bis 3, **dadurch gekennzeichnet,** daß die Polarisation der Katalysatorelektrode (2) im Elektrolytraum (5) mit einem Oxidationsmittel wie Sauerstoff, Wasserstoffperioxid, Perverbindungen, Chlor, Salpetersäure, Eisen-III-Salzen, Cer-III-salzen, Chrom-VI-salzen oder nitrosen Gasen erfolgt.

5. Biologischer Reaktor nach Anspruch 1 bis 3, **dadurch gekennzeichnet,** daß die Polarisation der Katalysatorelektrode (2) im Elektrolytraum (5) mit einer äußeren Spannungsquelle (6) über eine Hilfskathode (7) erfolgt.

6. Biologischer Reaktor nach Anspruch 1 bis 4, **dadurch gekennzeichnet,** daß die Polarisation der Elektroden (1, 2) im Reaktor- (4) und Elektrolytraum (5) über eine äußere Spannungsquelle (6) erfolgt.

7. Biologischer Reaktor nach Anspruch 1 bis 6, **dadurch gekennzeichnet,** daß die Potentialeinstellung der Katalysatorelektrode (2) zusätzlich über den pH-Wert im Elektrolytraum (5) reguliert wird.

8. Biologischer Reaktor nach Anspruch 1 bis 7, **dadurch gekennzeichnet,** daß der Polyelektrolyt (3) aus heterogenen oder homogenen Kationen- oder Anionenaustauschermembranen besteht.

9. Biologischer Reaktor nach Anspruch 1 bis 8, **dadurch gekennzeichnet,** daß die Elektroden (1, 2) ionendurchlässig sind und die Form von Netzen, Filzen, Streckmetallen, Sinterkörpern, Stäben, Lochblechen oder dergleichen aufweisen, wobei zumindest die polarisierte Elektrode (1) oberflächlich mit anoxidiertem, elektrisch leitendem Kohlenstoffmaterial beschichtet ist.

10. Biologischer Reaktor nach Anspruch 1 bis 9, **dadurch gekennzeichnet,** daß die Poren der Lochstellen der Elektroden (1, 2) zumindest teilweise mit Polyelektrolyt (3) ausgefüllt sind.

11. Biologischer Reaktor nach Anspruch 1 bis 10, **dadurch gekennzeichnet,** daß auf der anodisch polarisierten Elektrode (1) im Reaktorraum (4) Mikroorganismen und/oder Enzyme oberflächlich immobilisiert sind.

12. Biologischer Reaktor nach Anspruch 1 bis 11, **dadurch gekennzeichnet,** daß die Katalysatorelektrode (2) im Elektrolytraum (5) aus oberflächlich anoxidiertem Graphit besteht, der mit Molybdän- und/oder Wolfram-VI-Oxiden sowie Alkalisulfiden oder Molybdän und Iodaten dotiert ist.

13. Biologischer Reaktor nach Anspruch 1 bis 11, **dadurch gekennzeichnet,** daß die Katalysatorelektrode (2) im Elektrolytraum (5) oberflächlich mit sauerstoffübertragenden Metallchelaten sowie einem ionen- und elektronenleitenden oder ionen- und elektronenleitende Substanzen enthaltenden Bindemittel dotiert ist.

14. Biologischer Reaktor nach Anspruch 1 bis 11, **dadurch gekennzeichnet,** daß die Katalysatorelektrode (2) im Elektrolytraum (5) aus oberflächlich anoxidiertem Graphit besteht, der mit Vanadium-IV/V- und/oder Eisen-III- und/oder Titan-III-/Titan-IV-Verbindungen dotiert ist.

**Claims**

1. A biological reactor which is operated using micro-organisms and/or enzymes and used for the oxidative conversion of organic compounds, and in which the organic compounds to be converted are employed in the presence of a biocatalyst in the reactor space (4) (anode space) and the enzymes are regenerated at a polarized electrode (1), the polarization of the electrode (1) being effected by means of a catalyst electrode (2) which is polarized by an oxidizing agent, separated from the reactor space (4) by a polyelectrolyte (3) and located in an electrolyte space (5), wherein the electrode (1) is prepared from an electrically conductive carbon material which is partially oxidized on the surface at a potential $\epsilon_h$ of from +1.3 to +10 V in an aqueous oxygen-containing mineral acid.

2. A biological reactor as claimed in claim 1, wherein the anodically polarized electrode (1) is fixed to a polyelectrolyte (3) (ion exchanger) which separates the reactor space (4) from the electrolyte space (5), and a second polarizable catalyst electrode (2) which is permeable to ions and is electrically conductive or is connected to the anodically polarized electrode (1) in the reactor space (4) via an external voltage source (6) is located on or near the surface of the polyelectrolyte (3).

3. A biological reactor as claimed in claim 1 and 2, wherein the anodically polarized electrode (1) consists of graphite, graphite wool or active carbon, which has been subjected to partial electrochemical oxidation in aqueous nitric acid and then activated by cathodic reduction in a mineral acid.

4. A biological reactor as claimed in claims 1 to 3, wherein the polarization of the catalyst electrode (2) in the electrolyte space (5) is effected by means of an oxidizing agent, such as oxygen, hydrogen peroxide, a per compound, chlorine, nitric acid, an iron(III) salt, a cerium-(III) salt, a chromium(VI) salt or nitrous gases.

5. A biological reactor as claimed in claims 1 to 3, wherein the polarization of the catalyst electrode (2) in the electrolyte space (5) is effected by means of an external voltage source (6) and an auxiliary cathode (7).

6. A biological reactor as claimed in claims 1 to 4, wherein the polarization of the electrodes (1, 2) in the reactor space (4) and the electrolyte space (5) is effected via an external source (6).

7. A biological reactor as claimed in claims 1 to 6, wherein the potential setting for the catalyst electrode (2) is additionally controlled via the pH in the electrolyte space (5).

8. A biological reactor as claimed in claims 1 to 7, wherein the polyelectrolyte (3) consists of heterogeneous or homogeneous cation exchange or anion exchange membranes.

9. A biological reactor as claimed in claims 1 to 8, wherein the electrodes (1, 2) are permeable to ions and are in the form of nets, felts, expanded metals, sintered elements, rods, perforated plates or the like, at least the polarized electrode (1) being coated on the surface with partially oxidized, electrically conductive carbon material.

10. A biological reactor as claimed in claims 1 to 9, wherein the pores or holes of the electrodes (1, 2) are partially or completely filled with polyelectrolyte (3).

11. A biological reactor as claimed in claims 1 to 10, wherein micro-organisms and/or enzymes are immobilized on the surface of the anodically polarized electrode (1) in the reactor space (4).

12. A biological reactor as claimed in claims 1 to 11, wherein the catalyst electrode (2) in the electrolyte space (5) consists of graphite which is partially oxidized on the surface and is doped with molybdenum and/or tungsten(VI) oxides and alkali metal sulfides or molybdenum-(VI) oxides and iodates.

13. A biological reactor as claimed in claims 1 to 11, wherein the catalyst electrode (2) in the electrolyte space (5) is doped on the surface with oxygen-carrying metal chelates and a binder which is ion-conducting and electron-conducting or contains ion-conducting and electron-conducting substances.

14. A biological reactor as claimed in claims 1 to 11, wherein the catalyst electrode (2) in the electrolyte space (5) consists of graphite which is partially oxidized on the surface and doped with vanadium(IV)/(V) and/or iron(III) and/or titanium(III)/titanium(IV) compounds.

**Revendications**

1. Réacteur biologique fonctionnant avec des micro-organismes et/ou des enzymes pour la transformation par oxydation de composés organiques, avec lequel on met les composés organiques à transformer, en présence de biocatalyseurs, dans l'espace intérieur du réacteur (4) (espace anodique) et on régénère les enzymes à une électrode polarisée (1), la polarisation de cette électrode (1) étant effectuée par l'intermédiaire d'une électrode catalyseur (2) se trouvant dans un espace à électrolyte (5), séparée de l'espace intérieur du réacteur (4) par un polyélectrolyte (3) et polarisée par un oxydant, caractérisé par le fait que l'électrode (1) est faite d'une matière carbonée conductrice légèrement oxydée superficiellement à des niveaux de potentiel $_h$ de + 1,3 V à + 10 V dans des acides minéraux aqueux contenant de l'oxygène.

2. Réacteur biologique selon la revendication 1, caractérisé par le fait que l'électrode polarisée anodiquement (1) est fixée sur un polyélectrolyte (3) (échangeur d'ions) qui sépare l'espace intérieur du réacteur (4) de l'espace à électrolyte (5), et sur la surface ou à proximité de la surface de ce polyélectrolyte (3) se trouve une électrode catalyseur polarisable (2) perméable aux ions, qui est reliée par conduction électrique ou par l'intermédiaire d'une source extérieure de tension (6) à l'électrode polarisée anodiquement (1) située dans l'espace intérieur du réacteur (4).

3. Réacteur biologique selon les revendications 1 et 2, caractérisé par le fait que l'électrode polarisée anodiquement (1) est constituée de graphite, de laine de graphite ou de charbon actif qui a été légèrement oxydé électrochimiquement dans l'acide nitrique aqueux et ensuite activé par réduction cathodique dans des acides minéraux.

4. Réacteur biologique selon les revendications 1 à 3, caractérisé par le fait que la polarisation de l'électrode catalyseur (2) située dans l'espace à électrolyte (5) est effectuée avec un oxydant tel qu'oxygène, peroxyde d'hydrogène, percomposés, chlore, acide nitrique, sels de fer (III), sels de cérium (III), sels de chrome (VI) ou gaz nitreux.

5. Réacteur biologique selon les revendications 1 à 3, caractérisé par le effectuée que la polarisation de l'électrode catalyseur (2) située dans l'espace à électrolyte (5) est faite avec une source extérieure de tension (6) par l'intermédiaire d'une cathode auxiliaire (7).

6. Réacteur biologique selon les revendications 1 à 4, caractérisé par le fait que la polarisation des électrodes (1, 2) situées dans l'espace intérieur du réacteur (4) et l'espace à électrolyte (5) est effectuée par l'intermédiaire d'une source extérieure de tension (6).

7. Réacteur biologique selon les revendications 1 à 6, caractérisé par le fait que le réglage de potentiel de l'électrode catalyseur (2) est en plus régulé par l'intermédiaire du pH dans l'espace à électrolyte (5).

8. Réacteur biologique selon les revendications 1 à 7, caractérisé par le fait que le polyélectrolyte (3) est constitué de membranes échangeuses de cations ou d'anions hétérogènes ou homogènes.

9. Réacteur biologique selon les revendications 1 à 8, caractérisé par le fait que les électrodes (1, 2) sont perméables aux ions et ont la forme de filets, feutres, métaux déployés, corps frittés, barres, tôles perforées ou une forme semblable, l'électrode polarisée au moins (1) étant revêtue superficiellement de matière carbonée conductrice légèrement oxydée.

10. Réacteur biologique selon les revendications 1 à 9, caractérisé par le fait que les pores des endroits troués des électrodes (1, 2) sont remplis au moins partiellement de polyélectrolyte (3).

11. Réacteur biologique selon les revendications 1 à 10, caractérisé par le fait que des micro-organismes et/ou des enzymes sont immobilisés superficiellement sur l'électrode polarisée anodiquement (1) située dans l'espace de réacteur (4).

12. Réacteur biologique selon les revendications 1 à 11, caractérisé par le fait que l'électrode catalyseur (2) située dans l'espace à électrolyte (5) est constituée de graphite légèrement oxydé superficiellement qui est dopé avec des oxydes de molybdène (VI) et/ou de tungstène (VI) ainsi que des sulfures alcalins ou du molybdène et des iodates.

13. Réacteur biologique selon les revendications 1 à 11, caractérisé par le fait que l'électrode catalyseur (2) située dans l'espace à électrolyte (5) est dopée superficiellement avec des chélates métalliques transmettant de l'oxygène ainsi qu'un liant conducteur d'ions et d'électrons ou contenant des substances conductrices d'ions et d'électrons.

14. Réacteur biologique selon les revendications 1 et 11, caractérisé par le fait que l'électrode catalyseur (2) située dans l'espace' à électrolyte (5) est constituée de graphite légèrement oxydé superficiellement qui est dopé avec des composés de vanadium (IV/V) et/ou de fer (III) et/ou de titane (III)/titane (IV).

$C_2H_4O$

$H_2O$

1

3

2 NADH → 2 $H^+$

2 $NAD^+$

2 $OH^-$

ADH

$H_2O^-$

$C_2H_4O$ $C_2H_5OH$

2

5

4

$C_2H_5OH$

$1/2 \ O_2$

PH = 0 BIS 14

FIG.1

$C_2H_4O$

6

+ — 

1

2

2 NADH → 

3

2 NAD$^+$ 

4

2 H$^+$

ADH

2 OH– H$_2$

5

$C_2H_4O$ $C_2H_5OH$

2H$_2$O

7

$C_2H_5OH$

PH = 0 BIS 14

FIG.2

FIG.3

FIG.4

FIG.5

FIG.6